Europäisches Patentamt

European Patent Office

Office européen des brevets

(19)

(11) Publication number: **0 313 569 B1**

# (12) EUROPEAN PATENT SPECIFICATION

(45) Date of publication of patent specification: **18.11.93**

(51) Int. Cl.5: **A61K 35/26**, A61K 37/00, A61K 37/04, A61K 39/00, A61K 39/395, A61K 39/385, A61K 45/05

(21) Application number: **87904610.0**

(22) Date of filing: **07.07.87**

(86) International application number:
**PCT/US87/01559**

(87) International publication number:
**WO 88/00053 (14.01.88 88/02)**

(54) **ENHANCEMENT OF THE CELLULAR IMMUNE RESPONSE.**

(30) Priority: **08.07.86 US 883266**

(43) Date of publication of application:
**03.05.89 Bulletin 89/18**

(45) Publication of the grant of the patent:
**18.11.93 Bulletin 93/46**

(84) Designated Contracting States:
**AT BE CH DE FR GB IT LI LU NL SE**

(56) References cited:
**GB-B-21 069 35**
**US-A- 4 146 603**
**US-A- 4 356 170**
**US-A- 4 599 230**

**Science, 224: 1198, 15 June 1984, (Springer), P1199, 1200, 1201, 1202**

**Carbohydrate Research, 93, 35-41, (1981), (Ratcliffe et al) p.35, 36, 37, 38**

(73) Proprietor: **LONGENECKER, Bryan Michael**
**8412-118th Street**
**Edmonton, Alberta T6G 1T3(CA)**

Proprietor: **HENNINGSSON, Carina**
**8510-111 Street**
**Apt. 1808**
**Edmonton, Alberta T6G 1M7(CA)**

(72) Inventor: **LONGENECKER, Bryan Michael**
**8412-118th Street**
**Edmonton, Alberta T6G 1T3(CA)**
Inventor: **HENNINGSSON, Carina**
**8510-111 Street**
**Apt. 1808**
**Edmonton, Alberta T6G 1M7(CA)**

(74) Representative: **Patentanwälte Grünecker, Kinkeldey, Stockmair & Partner**
**Maximilianstrasse 58**
**D-80538 München (DE)**

Rank Xerox (UK) Business Services
(3.10/3.6/3.3.1)

Benjamini et al, "Basic and Clinical Immunology," Published 1984, 5th Edition, Stites et al, Eds, Chapter 15 p. 227, 228, 232

Cellular Immunology, 62, 38-49, (1981), Ertl, p. 38-40, 47

Chemical Abstracts, vol. 78, 27830Z, (1973), Columbus, Ohio, USA, Cooper, M.G.

Immunology, 41, 635, (1980), Lefford, MJ. p. 635

**Description**

BACKGROUND OF THE INVENTION

Vertebrates have two basic immune responses: humoral or cellular. Humoral immunity is provided by the special class of cells produced by B lymphocytes. These cells produce antibodies which circulate in the blood and lymphatic fluid. On the other hand, cell mediated immunity is provided by the T cells of the lymphatic system.

The cellular immune response is particularly effective against fungi, parasites, intracellular viral infections, cancer cells and foreign matter, whereas the humoral response primarily defends against the extracellular phases of bacterial and viral infections.

Containment of antigen at its point of entry is accomplished by walling off the area by local inflammation. Acute inflammation is characterized by the influx of plasma proteins and polymorphonuclear leukocytes. Chronic inflammation is characterized by the infiltration of T-lymphocytes and macrophages. When acute (antibody induced) and chronic (T-cell induced) inflammations occur in the skin, they are called immediate and delayed type hypersensitivity reactions respectively. ITH peaks at 24 hours, and subsides in 48 hours DTH appears in 24-48 hours and peaks at 48-72 hours. The subset of T cells involved in DTH reactions are called here DTH-Effector cells.

Epiglycanin (epi) is the major cell surface glycoprotein produced by the mammary adenocarcinoma transplantable cell line TA3Ha. Friberg, Jr., J.N.C.I., 48:1463 (1972); Codington, et al., Canc. Res., 43:4373 (1983). The TA3Ha carcinoma cells are covered by a mucin-like glycocalix composed mainly of epiglycanin. Codington, et al., J.N.C.I., 60:811 (1978); Miller, et al., J.N.C.I., 68:981 (1982). Epi is mainly carbohydrate in composition, and expresses multiple T and Tn determinants. T and Tn are general carcinoma autoantigens. Springer, Science, 224:1198 (1984).

Synthetic T and Tn antigens have been prepared, and used in DTH diagnostics. Lemieux, Ep Appl. 44,188. However, their therapeutic or prophylactic use has not been disclosed.

Bretscher, Eur. J. Immunol., 9:311-316 (1979) cultured DTH effector cells in vitro and injected them with the sensitizing antigen (burro erythrocytes) into the foot pads of mice. Ertl, Cellular Immunology, 62:38-49 (1981) investigated antigenic and genetic requirements for induction and effector function of Td lymphocytes directed against Sendai virus.

In GB-A-2 106 935 a method is described to produce killer cells which act on cancer cells having a glyco-related antigen derived from the cancer cells.

We have used cancer-associated, well characterized carbohydrate antigens to sensitize effector cells and have taught a therapeutic use for them.

It is known that tumors may be treated with a mixture of IL-2 and IL-2-activated killer cells. See Fortune, 16-21 (November 25, 1985), reporting on the work of Steven Rosenberg. The problem with the Rosenberg work is that the therapy induces a population of cells known as LAK (lymphokine activated killer) cells. LAK cells are not antigen-specific; therefore normal tissue may be attacked. Also, since the LAK cells are nonspecific, relatively large doses of IL-2 are required to induce the required anti-tumor activity. Finally,. LAK cells are not thought to confer memory to the immune system. In contrast, DTH effector cells are antigen specific; therefore after stimulation much smaller quantities of lymphokine may be required to induce proliferation. Also because of their specificity, DTH effector cells are less likely to attack self. Moreover, DTH effector cells are thought to have memory.

SUMMARY OF THE INVENTION

We have found that the cell-mediated immune response to cancer cells is enhanced by parenteral administration of natural and synthetic cancer-associated carbohydrate antigens or by parenteral administration of DTH effector cells primed by such antigens. This enhancement is likely to be advantageous for both therapy and prophylaxis when the body's primary defense against a challenge is a cellular immune response, e.g., against tumors. The use of other specific carbohydrate antigens, including polysaccharides, glycolipids and glycoproteins, may be useful in protecting a subject against other threats controlled by T cells. It is known that parasites, viruses, bacteria and fungi bear surface carbohydrate determinants.

Lymphokines may be used in conjunction with the specific antigens or primed DTH effector cells of this invention to enhance the specific CMI response.

## BRIEF DESCRIPTION OF THE DRAWINGS

Fig. 1 shows that epi-primed splenocytes induce local DTH swellings in the presence of epi, while unprimed spleen cells, irradiated tumor cells, and primed cells in the absence of antigen do not.

Fig. 2 shows that this reaction is specific to cancer cell lines bearing the primer as a surface antigen.

Fig. 3 shows that the reaction was with the T alpha, T beta and Tn determinants of epi.

Fig. 4 shows that the phenotype of the anti-carbohydrate DTH effector cells is Thy-1 +, Lyt-1 +, Lyt-2-.

Fig. 5 shows systemic transfer of DTH by a DTH reaction to irradiated tumor cells after IV administration of Effectors.

Fig. 6 shows inhibition of tumor growth after local transfer of DTH effector cells mixed with tumor cells.

Fig. 7 shows the importance of the priming dose.

Fig. 8 shows that at lower doses of antigen the cellular response predominated.

## DETAILED DESCRIPTION OF THE INVENTION

### Example 1

Use of S-TAGs to Enhance Cellular Immune Response to Tumors

Synthetic tumor-associated glycoproteins (S-TAGs) and other carbohydrate antigens are known in the art and may be prepared by any convenient technique. T and Tn antigens are preferred. For synthetic methods, see Kaifu and Osawa, Carbohydr. Res., 58:235 (1977); Ratcliffe, et al., 93:35 (1981); Paulsen, et al., 104:195 (1982); Bencomo and Sinay, 116:69 (1983).

Soluble S-TAGs or their aggregates are mixed with an adjuvant for intradermal, intramuscular or intraperitoneal administration. It is conceivable that intravenous administration will also be successful.

Groups of five mice at a time were primed with specific doses of T-alpha (TF), T-beta (asialo-GM1 disaccharide) and Tn S-TAGs. After 7-10 days, they were challenged with $3 \times 10^3$ live TA3Ha cells. Markedly increased survival was noted in mice receiving about 1 ug of synthetic antigen. Thus, for T-beta, the following results were observed (Table I):

Table I

| Dose | Survival (days) |
|------|-----------------|
| 0.2 ug | 20, 27, 27, 29, 31 |
| 1.0 ug | 27, 38, 43, 43, 49 |
| 2.0 ug | 16, 21, 24, 30 |
| 10 ug | 20, 21, 28, 33 |
| 20 ug | 21, 21, 28, 28 |
| 50 ug | 16, 18, 31 |
| 100 ug | 20, 20, 21, 27, 29 |
| PBS | 23, 27, 29 |
| Norm | 13, 13, 18, 27, 28 |

It should be noted that TA3Ha is very deadly. The normal post-transplantation life expectancy of a mouse is only 15-20 days.

### Example 2

Use of Cultured DTH Effector Cells as Tumor Inhibitors

We obtained mouse primed DTH effector cells by the following procedure. CAF1/J (Balb x A/J) mice from our animal unit were immunized with 30 ug epi (or 2 ug of any synthetic antigen), in 50% complete Freund's adjuvant, intraperitoneally, boosted one week later with a similar injection. 7-10 days after the last injection the animals were sacrificed, their spleens removed and passed through a nylon mesh to make a single cell suspension. The cells were washed and cultured in a costar well at $1.5 \times 10^7$ cells/8ml. (RPMI + P.S. + 10% FCS)/well. Each well received 10 ug epi (or 1-2 ug of any of the synthetic antigens). The cells were cultured at 37° C and 5% $CO_2$ for 6 days and then harvested by gently dissociating them from the plastic with a rubber policeman. The primed cells were then tested for DTH reactivity with a number of antigens, and for their ability to inhibit the growth of TA3Ha cell line in vivo.

To obtain human primed DTH effector cells, we would use a modification of the method of Rosenberg. Lymphocytes are removed from blood of a living subject by plasmapheresis. The lymphocytes are cultured with a specific antigen, such as cancer-associated glycoprotein (epiglycanin or a T or Tn S-TAG) or the combination of a specific antigen with IL-2, rather than with IL-2 alone. The human lymphocytes are cultured in suitable media for 6 days at a preferred concentration of about $10^6$ cells/ml. The cells are washed to remove antigen. The cells may then be returned to the patient, intravenously, for therapeutic or prophylactic purposes.

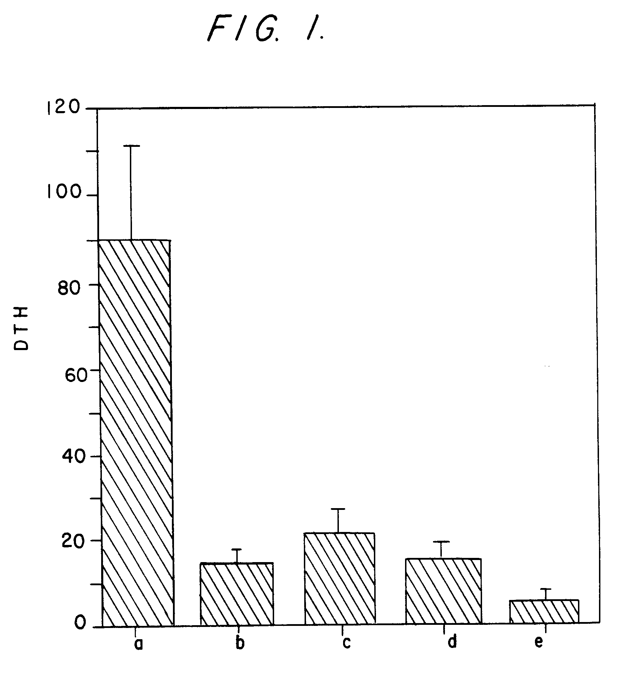

We have demonstrated that co-injection of a mammal with epiglycanin-primed DTH effector cells and antigen (irradiated tumor cells) induced a DTH reaction (Figure 1). Spleen cells from mice primed and boosted with 30 ug and 20 ug of epi extracted and purified from TA3Ha ascites fluid and CFA (intraperitoneally administered one and two weeks beforehand) were cultured at $1.5 \times 10^7$ cells/well with: (a) 10 ug epi/well and (b) no antigen. In addition, unprimed spleen cells were cultured with (c) 10 ug epi/well and (d) no antigen. Cultures were harvested on the 6th day and $10^7$ cells were injected subcutaneously into the foot pad of unimmunized mice together with $10^6$ irradiated TA3Ha cells. As a control (e), $10^6$ irradiated TA3Ha cells were injected alone. All the DTH values are presented as net DTH swellings, i.e., [swelling of primed cells + Ag.] - [swelling of primed cells alone]. The large differential responses demonstrated that immunization with a cancer mucin can induce a DTH reaction to those cancer cells which have the mucin at the cell surface.

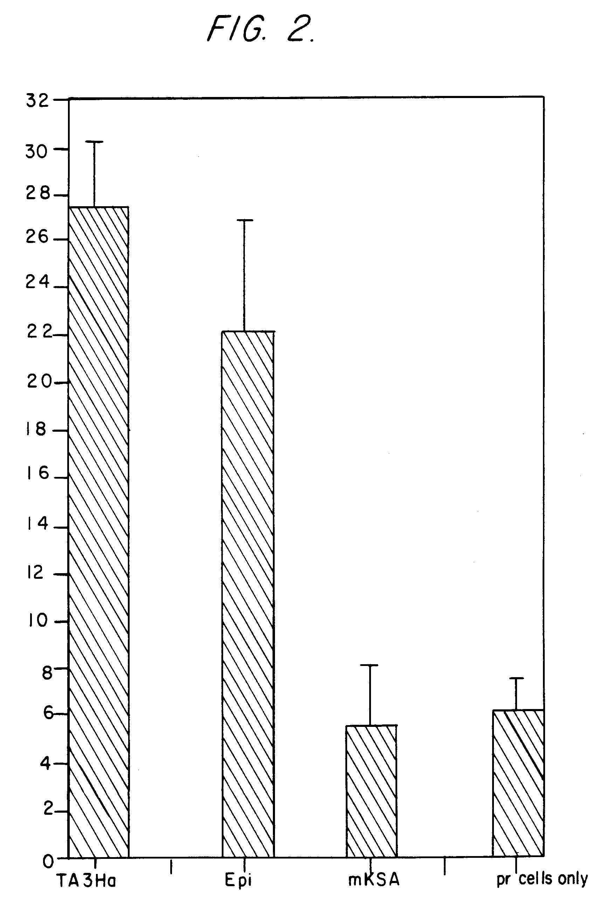

Subsequently, we showed that this reaction was tumor-specific (Fig 2). A DTH effector cell population was generated as in Example I and tested with a) TA3Ha; b) Epi-M (epi linked to a sepharose microsphere); c) mKSA; and d) no antigen. All the DTH values are presented as net DTH swellings, i.e., [swelling of primed cells + Ag.] - [swelling of primed cells alone]. The result was that the immunization with the epi mucin induced a specific DTH response to the mucin and to the cells which bear the mucin (TA3Ha) but not against those that do not bear the mucin (mKSA).

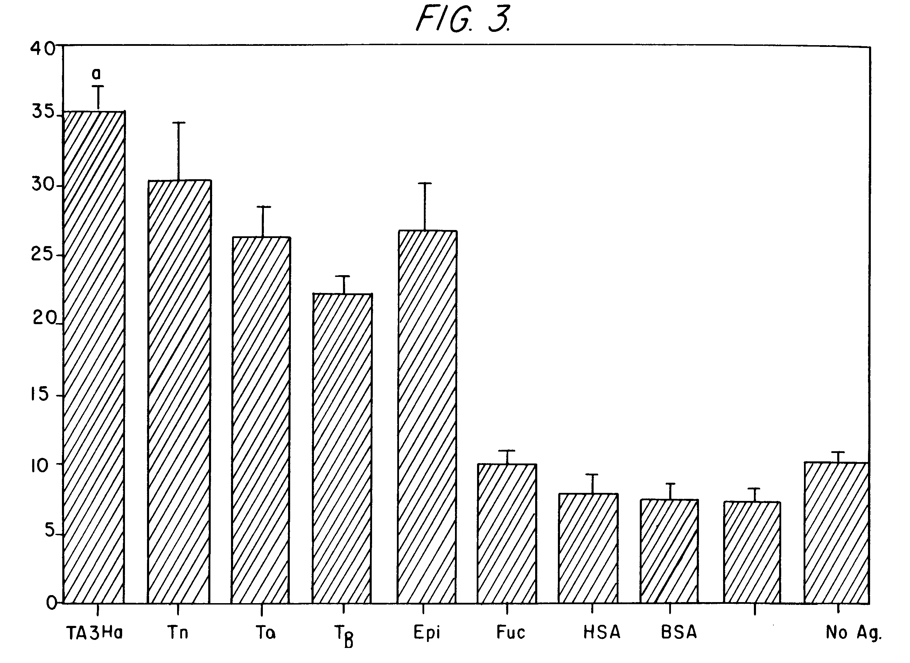

We also explored the antigenic specificity of the DTH reaction in greater detail (Fig. 3). $10^7$ T cells were primed with epi and were co-injected with a series of antigens (TA3Ha, Tn, T-alpha, T-beta, epi, Fucose ("Fuc"), HSA, BSA, sepharose microspheres, and no antigen). All soluble antigens were linked to sepharose microspheres and used at 12 ug/foot pad except epi which was used at 50 ug/foot pad. All DTH results are net DTH measurements. The experiment shows that the DTH measured by the injection of epi reacts with the carbohydrate determinants on S-TAGs which are also known to be epi determinants but not with those carbohydrates determinants not represented on epi. (eg. Fucose).

## SUMMARY OF SPECIFICITY OF DTH RESPONSE

| IMMUNIZING Ag: | TRIGGERING Ag: | | | | | |
|---|---|---|---|---|---|---|
| | Epi | Tn | Ta | $T_b$ | Fuc | BSA |
| Epi: | + | + | + | + | - | - |
| $T_a$: | + | + | + | + | - | - |
| $T_B$: | + | + | + | + | - | - |
| Tn: | + | + | + | + | - | - |
| None: | - | - | ND | ND | ND | |

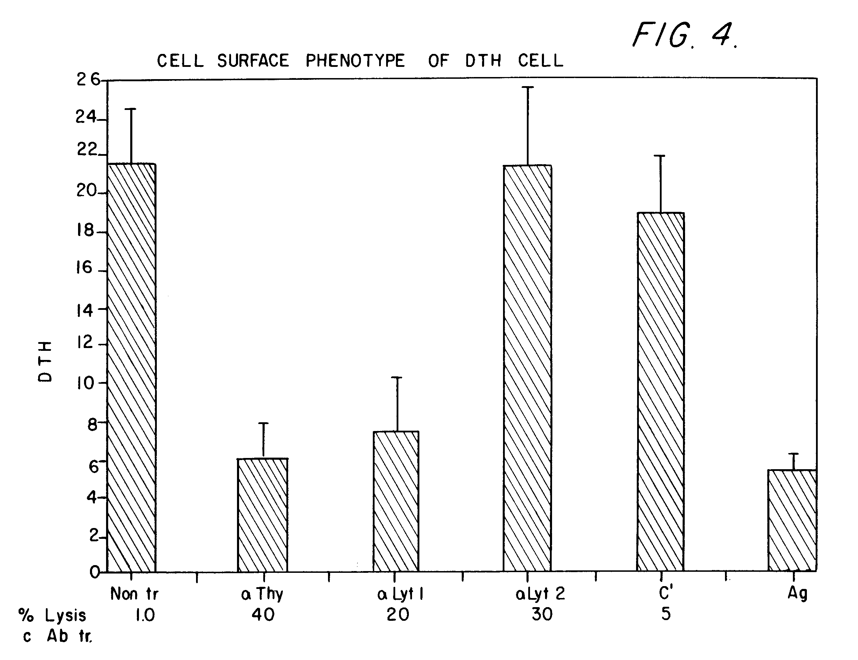

To confirm that the reactions we were witnessing were indeed DTH reactions we designed an experiment to check for the cell surface phenotype of DTH effector cells. (Fig. 4) An effector cell population was prepared as before and treated with a) no treatment; b) anti-Thy 1.2 antibodies plus complement; c) anti-Lyt 1.2 plus complement; d) anti-Lyt 2.2 plus complement; and e) complement alone. The antigen alone was also used as a control. All the values presented are net DTH. Treatment of the DTH effector cells with anti-Thy or anti-Lyt-1 antibodies with complement eliminate the DTH effector cell activity but treatment with anti-Lyt-2 plus complement did not eliminate the DTH effector cell activity; thus, the phenotype of the specific anti-carbohydrate DTH effector cells is Thy-1 +, Lyt-1 +, Lyt-2⁻---the phenotypic pattern shown by other DTH effector cells reacting against proteins. See Fujiwara, J. Immunol, 135:2187 (September, 1985).

The protocol for this comparison was as follows. A primed cell population was obtained, washed, counted, and divided into separate tubes for treatment. The cells were gently pelleted and resuspended in Leibowitz media containing 1) anti Thy 1.2 antibody (at 1/1000 diln., obtained from NEN), 2) anti Lyt 1.2 antibody (at 1/1000 diln., obtained from NEN) or 3) anti Lyt 2.2 antibody (at 1/1000 diln., obtained from NEN) at $2 \times 10^7$ cells/ml., and incubated at 4°C for 45 min. The cells were then spun down and resuspended in guinea pig complement (1/10 diln.) in Leibowitz media at $2 \times 10^7$ cells/ml., and incubated for 30 min. in a 37°C water bath. After incubation, cells were washed with Leib + 10% FCS and counted.

A comparative study was undertaken to determine whether the reaction was of the delayed type. DTH effector cells were injected together with TA3Ha and the reaction was compared with reactions in other mice injected with only the DTH effector cells or only the TA3Ha. It was found that the reaction peaked in 24 hours and had disappeared by 96 hours. No DTH (emphasis on delayed) was found with the two controls.

As further confirmation that the reaction observed was a DTH, a mononuclear infiltrate was observed the site of swelling.

The foregoing experiments all related to local transfer of DTH. We also investigated the possibility that DTH effector cells would have a systemic effect.

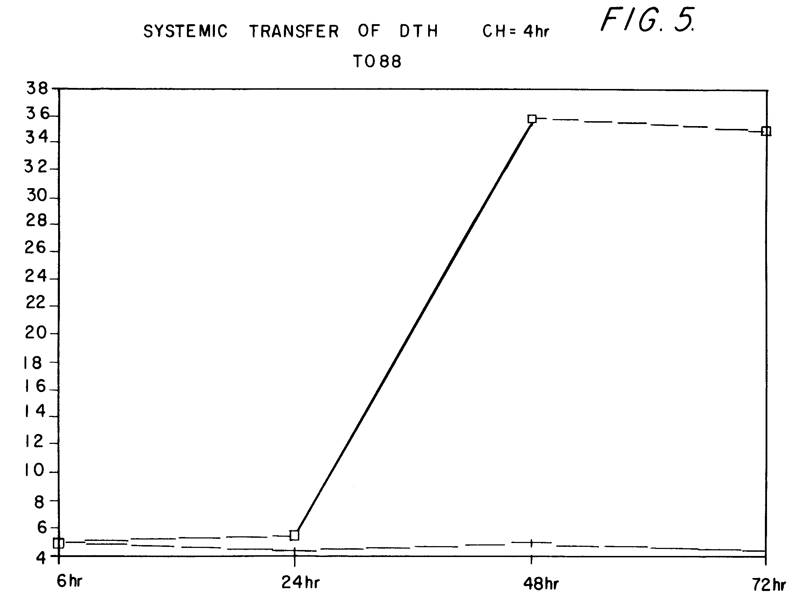

DTH effector cells were administered intravenously to the host animal. Four hours later, irradiated TA3Ha cells were injected into the footpad. At 6, 24, 48 and 72 hours, the DTH reaction was measured (open boxes) and compared with a negative control (plusses (Fig. 5).

The irradiated tumor cells used in the above-described experiments will not proliferate. In the next experiments, however, untreated tumor cells were employed.

DTH primed cells were induced as described above. $5 \times 10^7$ primed cells injected IV plus $2.5 \times 10^5$ live tumor cells were injected s.c. into the foot pad. The control mice were injected with normal spleen cells plus tumour cells. The foot pad swelling was typically measured at 12, 24, 48, and 72 hours and every two days thereafter. The determine the component of the swelling which was due to a DTH reaction, as opposed to a combination of DTH and tumour growth, mice were injected with primed or normal cells plus irradiated tumor cells. The swelling was attributed only to the DTH reaction. Tumor growth was measured every 2 days starting 48 hours after the peak DTH swelling. The DTH and the tumor size were measured using calipers in order to estimate the increase in foot pad thickness.

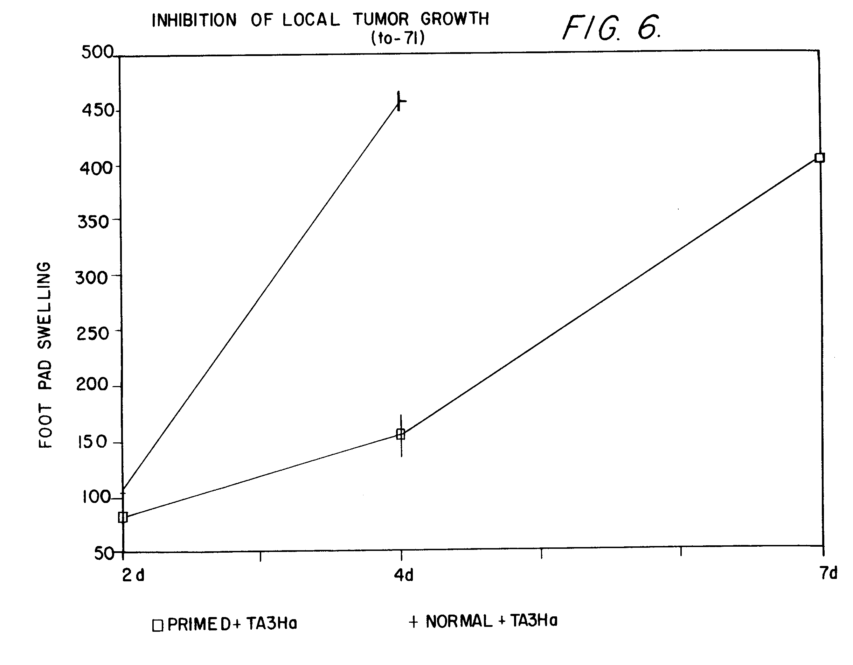

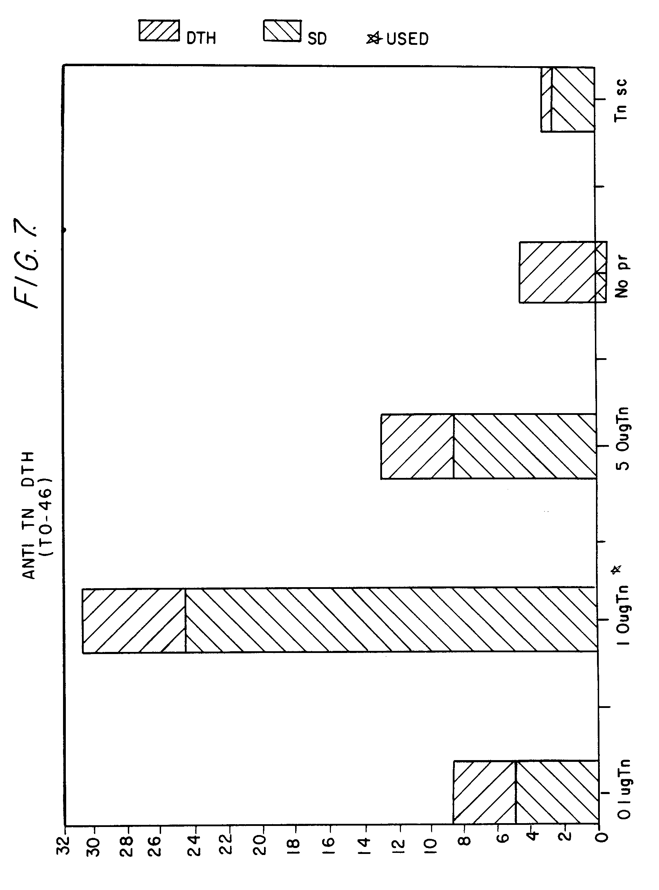

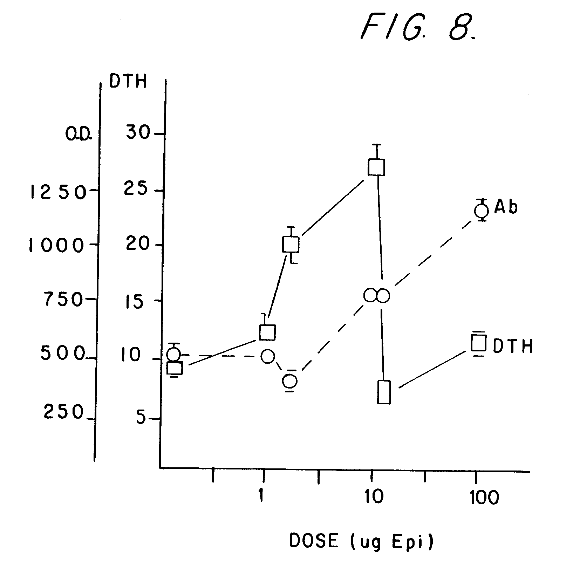

Fig. 6 shows that the primed cells inhibited the growth of the TA3Ha tumor. (Open boxes, primed DTH effector cell plus TA3Ha; pluses, normal cells plus TA3Ha.

Other experiments have shown that the nature of the reaction to the antigen is dependent on the dose of primer administered to the DTH effector cells. At lower doses, cell-mediated immunity was predominant, while at higher doses, the reverse was true.

A dose dependence was also found when splenocytes are primed with Tn in culture (Fig. 7). (Rising shading bar shows net DTH; Falling shading bar shows standard deviation.)

We have further shown that the DTH effector cells stimulated by epi will "home in" on a tumor and inhibit tumor growth. DTH effector cells were primed with epi as previously described. One day prior to intravenous injection of the epi-primed DTH effector cells, the TA3Ha tumor was transplanted into the footpads of mice. Mice with tumors transplanted at the same time were used as controls and tumor growth was determined by measuring the welling of the footpads (in mm) with calipers.

| EXPERIMENT 1 | | |
|---|---|---|
| | Day 6. post iv | Day 8, post iv |
| Systemic Transfer Effector cells Control Animals (3 mice) | 10, 12, 12, 10, 15 140, 25, 35 | 50, 15, 80, 80, 50 190, 200, 220 |

| EXPERIMENT 2 | | | | |
|---|---|---|---|---|
| | Day 5 post iv | Day 6 post iv | Day 8 post iv | Day 10 post iv |
| Systemic Transfer of DTH Effector cells (4 mice) | 10,20,15,10 | 55,20,15,20 | 100,140,80,60 | 300,360,340 |
| Control Animals (4 mice) | 60,75,60,110 | 170,160,170 110 | 340,300,320, 340 | 500(3mice) |

As may be seen from the above data, the IV-administered, epi-primed DTH effector cells exerted a tumor-inhibitory effect at the footpad site of tumor transplantation.

## Example 3

Use of Epiglycanin as a Tumor Inhibitor

Epiglycanin (epi) was extracted from the ascites fluid of TA3Ha tumor-bearing mice.

Ascites from outbred TA3Ha ip tumors was collected and cells were removed by centrifugation. The samples were stored frozen. Before extraction, the ascites was thawed and incubated at 37° C for 2 hours, and any aggregates were removed by centrifugation. The freezing, thawing and clotting procedure was performed twice before PNA extraction.

25 - 40 ml. bed volume of PNA agarose (E. Y. Laboratories) was washed with PBS, mixed with ascites and tumbled gently at 4°C for 24 - 48 hours. The slurry was poured into a column and extensively washed with PBS at room temperature. Epi was eluted from PNA agarose using galactose. The eluted epi-galactose mixture was then dialyzed against water to remove the galactose. The sample was lyophilized and stored at -20°C. in a dessicator.

Groups of five mice at a time were primed with specific doses of epi. After 2-3 days they were challenged with $3 \times 10^3$ live TA3Ha cells. Table 2 below shows the relationship between the priming dose and survival.

**Table 2**

| Priming dose: | Survival: (days) | ave. | s.e. | No. alive (at d. 50) |
|---|---|---|---|---|
| 0.2 ug | 19, 25, 27, 49, 49 | 33.8 | 6.3 | 2/5 |
| 1 ug | 25. 49, 49, 49, 49 | 44.2 | 4.8 | 4/5 |
| 2 ug | 14, 19, 19, 25, 49 | 25.2 | 6.2 | 4/5 |
| 10 ug | 20, 22, 22, 25 | 22.3 | .9 | 0/4 |
| 20 ug | 20, 22, 25, 49, | 29.0 | 6.0 | 1/5 |
| 100 ug | 19, 19, 20, 25, 49 | 25.5 | 5.3 | 1/5 |
| PBS | 19, 19, 20, 20 | 20 | 0.3 | 0/4 |

Data calculated on day 49 i.e. these mice are still alive. These mice were sacrificed on

### day 88

to continue the experiment.

Thus, the preferred priming dose was 1 ug, and this dosage appeared to confer protective immunity against TA3Ha cells.

In another experiment, it was determined that the humoral response was negligible at that dosage (Fig. 8), suggesting that the tumor was inhibited by a mechanism other than antibodies, possibly CMI. Mice (CAF1/J) were injected with the indicated doses of epi i.p. in 50% CFA. Ten days later they were challenged with irradiated TA3Ha cells to determine the DTH reactivity (closed boxes). Antibody to epi (open circles) was determined by ELISA.

A summary of our experiences with epi and T-alpha in tumor inhibition is given in Table 3 below:

**Table 3:  In Vivo Protection;**
**Number of animals alive at 4 weeks.**

| Dose of Ag | Epi | Ta | Glu-BSA/HSA |
|---|---|---|---|
| 0.1 ug | 1/20 | 4/15 | 0/10 |
| 0/5 ug | 5/20 | 7/15 | 0/10 |
| 1.0 ug | 8/20 | 5/15 | 0/10 |
| 5.0 ug | 8/15 | 6/15 | 0/10 |
| 10.0 ug | 7/20 | 3/15 | 0/10 |
| cont. | 0/20 | 0/15 | 0/10 |

Since Glu-BSA/HSA presents a carbohydrate which is

**not**

expressed on the surface of the TA3Ha tumor cells, administration of that substance did not provide protection. Epi, which is the predominant surface carbohydrate antigen on TA3Ha cells, and T-alpha, which is one of the epi determinants, did provide protection.

**Claims**
**Claims for the following Contracting States : BE, CH, DE, FR, GB, IT, LI, LU, NL, SE**

1.   A method of priming T cells involved in delayed type hypersensitivity reactions (DTH effector cells), which are specific for a predetermined tumor-associated carbohydrate determinant to enhance their immunological activity in tumor inhibition, which comprises cultivating DTH effector cells in a medium containing a priming amount of a specific antigen bearing said carbohydrate determinant.

2.   The method of Claim 1 in which the antigen is a mucin.

3.   The method of Claim 2 in which the antigen is epiglycanin.

4.   The method of Claim 1 in which the antigen is a glycoprotein.

5.   The method of Claim 1 in which the antigen is a glycolipid.

6.   The method of Claim 1 in which the antigen bears a T-alpha determinant.

7.   The method of Claim 1 in which the antigen bears a T-beta determinant.

8.   The method of Claim 1 in which the antigen bears a Tn determinant.

9.   The method of Claim 1, wherein the antigen comprises a synthetic carbohydrate hapten conjugated to an immunological carrier.

10.  The method of Claim 9, wherein the hapten bears a determinant selected from the group consisting of T-alpha, T-beta and Tn determinants.

11.  The method of Claim 1 in which the antigen is a polysaccharide.

12.  The method of Claim 1 in which the medium further comprises a lymphokine.

13.  The method of Claim 12 in which the lymphokine is IL-2.

8

**14.** An essentially biologically pure culture of primed DTH effector cells specific for a predetermined tumor-associated carbohydrate determinant, wherein said effector cells have been primed by a method according to any of Claims 1 to 13.

**15.** A therapeutic composition comprising an effective amount of primed DTH effector cells specific for a predetermined tumor-associated carbohydrate determinant in a pharmaceutically acceptable carrier, wherein the DTH effector cells have been primed by a method according to any of Claims 1 to 13.

**16.** The composition of Claim 15 further comprising irradiated tumor cells.

**17.** A composition comprising an effective amount of a specific antigen bearing a tumor-associated carbohydrate determinant in a pharmaceutically acceptable carrier for use as a medicament.

**18.** The composition of Claim 17 in which the antigen is a mucin.

**19.** The composition of Claim 18 in which the mucin is epiglycanin.

**20.** The composition of Claim 17 in which the determinant is T-alpha.

**21.** The composition of Claim 17 in which the determinant is T-beta.

**22.** The composition of Claim 17 in which the determinant is Tn.

**23.** The composition of any of Claims 20 to 22 in which the antigen comprises a synthetic hapten conjugated to an immunological carrier.

**24.** Use of the culture of primed DTH effector cells according to Claim 14 for preparing a medicament for enhancing the cellular immunity in a mammal.

**25.** Use of a DTH effector cell priming amount of a carbohydrate antigen bearing a specific tumor-associated carbohydrate determinant for the preparation of a medicament for enhancing the cellular immunity in a mammal.

**26.** Use according to Claim 25, wherein the antigen is a mucin.

**27.** Use according to Claim 26, wherein the mucin is epiglycanin.

**28.** Use according to Claim 25, wherein the determinant is T-alpha.

**29.** Use according to Claim 25, wherein the determinant is T-beta.

**30.** Use according to Claim 25, wherein the determinant is Tn.

**31.** Use according to any of claims 28 to 30, wherein the antigen comprises a synthetic hapten conjugated to an immunological carrier.

**32.** Use of epiglycanin-primed DTH effector cells and irradiated tumor cells for the preparation of a medicament for inducing a DTH reaction in a mammal.

**Claims for the following Contracting State : AT**

**1.** A method of priming T cells involved in delayed type hypersensitivity reactions (DTH effector cells), which are specific for a predetermined tumor-associated carbohydrate determinant to enhance their immunological activity in tumor inhibition, which comprises cultivating DTH effector cells in a medium containing a priming amount of a specific antigen bearing said carbohydrate determinant.

**2.** The method of Claim 1 in which the antigen is a mucin.

9

**3.** The method of Claim 2 in which the antigen is epiglycanin.

**4.** The method of Claim 1 in which the antigen is a glycoprotein.

**5.** The method of Claim 1 in which the antigen is a glycolipid.

**6.** The method of Claim 1 in which the antigen bears a T-alpha determinant.

**7.** The method of Claim 1 in which the antigen bears a T-beta determinant.

**8.** The method of Claim 1 in which the antigen bears a Tn determinant.

**9.** The method of Claim 1, wherein the antigen comprises a synthetic carbohydrate hapten conjugated to an immunological carrier.

**10.** The method of Claim 9, wherein the hapten bears a determinant selected from the group consisting of T-alpha, T-beta and Tn determinants.

**11.** The method of Claim 1 in which the antigen is a polysaccharide.

**12.** The method of Claim 1 in which the medium further comprises a lymphokine.

**13.** The method of Claim 12 in which the lymphokine is IL-2.

**14.** An essentially biologically pure culture of primed DTH effector cells specific for a predetermined tumor-associated carbohydrate determinant, wherein said effector cells have been primed by a method according to any of Claims 1 to 13.

**15.** A method of preparing a therapeutic composition comprising an effective amount of primed DTH effector cells specific for a predetermined tumor-associated carbohydrate determinant in a pharmaceutically acceptable carrier wherein the DTH effector cells have been primed by a method according to any of Claims 1 to 13, which comprises mixing said primed DTH effector cells with said carrier in a manner known per se.

**16.** The method of Claim 15 in which irradiated tumor cells are additionally mixed to the composition.

**17.** A method of preparing a composition comprising an effective amount of a specific antigen bearing a tumor-associated carbohydrate determinant in a pharmaceuticallay acceptable carrier for use as a medicament, which comprises mixing said specific antigen with said carrier in a manner known per se.

**18.** The method of Claim 17 in which the antigen is a mucin.

**19.** The method of Claim 18 in which the mucin is epiglycanin.

**20.** The method of Claim 17 in which the determinant is T-alpha.

**21.** The method of Claim 17 in which the determinant is T-beta.

**22.** The method of Claim 17 in which the determinant is Tn.

**23.** The method of any of Claims 20 to 22 in which the antigen comprises a synthetic hapten conjugated to an immunological carrier.

**24.** Use of the culture of primed DTH effector cells according to Claim 14 for preparing a medicament for enhancing the cellular immunity in a mammal.

**25.** Use of a DTH effector cell priming amount of a carbohydrate antigen bearing a specific tumor-associated carbohydrate determinant for the preparation of a medicament for enhancing the cellular immunity in a mammal.

**26.** Use according to Claim 25, wherein the antigen is a mucin.

**27.** Use according to Claim 26, wherein the mucin is epiglycanin.

**28.** Use according to Claim 25, wherein the determinant is T-alpha.

**29.** Use according to Claim 25, wherein the determinant is T-beta.

**30.** Use according to Claim 25, wherein the determinant is Tn.

**31.** Use according to any of claims 28 to 30, wherein the antigen comprises a synthetic hapten conjugated to an immunological carrier.

**32.** Use of epiglycanin-primed DTH effector cells and irradiated tumor cells for the preparation of a medicament for inducing a DTH reaction in a mammal.

**Patentansprüche**

**Patentansprüche für folgende Vertragsstaaten : BE, CH, DE, FR, GB, IT, LI, LU, NL, SE**

**1.** Verfahren zum Sensibilisieren von,an verzögerten Überempfindlichkeitsreaktionen beteiligten T-Zellen (DTH-Effektorzellen), welche spezifisch für eine prädeterminierte, Tumor-assoziierte Kohlenhydratdeterminante sind, um deren immunologische Aktivität bei der Tumorhemmung zu steigern, welches das Kultivieren von DTH-Effektorzellen in einem Medium umfaßt, das eine sensibilisierende Menge eines spezifischen Antigens, welches die Kohlenhydratdeterminante trägt, enthält.

**2.** Verfahren nach Anspruch 1, in welchem das Antigen Mucin ist.

**3.** Verfahren nach Anspruch 2, in welchem das Antigen Epiglycanin ist.

**4.** Verfahren nach Anspruch 1, in welchem das Antigen ein Glycoprotein ist.

**5.** Verfahren nach Anspruch 1, in welchem das Antigen ein Glycolipid ist.

**6.** Verfahren nach Anspruch 1, in welchem das Antigen eine T-alpha-Determinante trägt.

**7.** Verfahren nach Anspruch 1, in welchem das Antigen eine T-beta-Determinante trägt.

**8.** Verfahren nach Anspruch 1, in welchem das Antigen eine Tn-Determinante trägt.

**9.** Verfahren nach Anspruch 1, worin das Antigen ein,an einen immunologischen Träger konjugiertes, synthetisches Kohlenhydrat-Hapten umfaßt.

**10.** Verfahren nach Anspruch 9, worin das Hapten eine Determinante, ausgewählt aus der Gruppe, bestehend aus T-alpha-, T-beta- und Tn-Determinanten, trägt.

**11.** Verfahren nach Anspruch 1, in welchem das Antigen ein Polysaccharid ist.

**12.** Verfahren nach Anspruch 1, in welchem das Medium weiterhin ein Lymphokin umfaßt.

**13.** Verfahren nach Anspruch 12, in welchem das Lymphokin IL-2 ist.

**14.** Eine im wesentlichen biologisch reine Kultur sensibilisierter DTH-Effektorzellen, die spezifisch für eine prädeterminierte, Tumor-assoziierte Kohlenhydratdeterminante sind, worin die Effektorzellen durch ein Verfahren gemäß einem der Ansprüche 1 bis 13 sensibilisiert worden sind.

**15.** Therapeutische Zusammensetzung, umfassend eine wirksame Menge sensibilisierter DTH-Effektorzellen, die spezifisch für eine prädeterminierte, Tumor-assoziierte Kohlenhydratdeterminante sind, in einem pharmazeutisch annehmbaren Träger, worin die DTH-Effektorzellen durch ein Verfahren gemäß einem der Ansprüche 1 bis 13 sensibilisiert worden sind.

**16.** Zusammensetzung nach Anspruch 15, weiterhin umfassend bestrahlte Tumorzellen.

**17.** Zusammensetzung, umfassend eine wirksame Menge eines spezifischen, eine Tumor-assoziierte Kohlenhydratdeterminante tragenden Antigens in einem pharmazeutisch annehmbaren Träger zur Verwendung als ein Medikament.

**18.** Zusammensetzung nach Anspruch 17, in welcher das Antigen ein Mucin ist.

**19.** Zusammensetzung nach Anspruch 18, in welcher das Mucin Epiglycanin ist.

**20.** Zusammensetzung nach Anspruch 17, in welcher die Determinante T-alpha ist.

**21.** Zusammensetzung nach Anspruch 17, in welcher die Determinante T-beta ist.

**22.** Zusammensetzung nach Anspruch 17, in welcher die Determinante Tn ist.

**23.** Zusammensetzung nach einem der Ansprüche 20 bis 22, in welcher das Antigen ein an einen immunologischen Träger konjugiertes, synthetisches Hapten umfaßt.

**24.** Verwendung der Kultur sensibilisierter DTH-Effektorzellen nach Anspruch 14 zum Herstellen eines Medikaments zum Verstärken der zellulären Immunität in einem Säuger.

**25.** Verwendung einer DTH-Effektorzellen sensibilisierenden Menge eines eine spezifische, Tumor-assoziierte Kohlenhydratdeterminante tragenden Kohlenhydratantigens für die Herstellung eines Medikaments zum Verstärken der zellulären Immunität in einem Säuger.

**26.** Verwendung nach Anspruch 25, worin das Antigen ein Mucin ist.

**27.** Verwendung nach Anspruch 26, worin das Mucin Epiglycanin ist.

**28.** Verwendung nach Anspruch 25, worin die Determinante T-alpha ist.

**29.** Verwendung nach Anspruch 25, worin die Determinante T-beta ist.

**30.** Verwendung nach Anspruch 25, worin die Determinante Tn ist.

**31.** Verwendung nach einem der Ansprüche 28 bis 30, worin das Antigen ein an einen immunologischen Träger konjugiertes, synthetisches Hapten umfaßt.

**32.** Verwendung von mit Epiglycanin sensibilisierten DTH-Effektorzellen und bestrahlten Tumorzellen für die Herstellung eines Medikaments zum Induzieren einer DTH-Reaktion in einem Säuger.

**Patenansprüche für folgenden Vertragsstaat : AT**

**1.** Verfahren zum Sensibilisieren von, an verzögerten Überempfindlichkeitsreaktionen beteiligten T-Zellen (DTH-Effektorzellen), welche spezifisch für eine prädeterminierte, Tumor-assoziierte Kohlenhydratdeterminante sind, um deren immunologische Aktivität bei der Tumorhemmung zu steigern, welches das Kultivieren von DTH-Effektorzellen in einem Medium umfaßt, das eine sensibilisierende Menge eines spezifischen Antigens, welches die Kohlenhydratdeterminante trägt, enthält.

**2.** Verfahren nach Anspruch 1, in welchem das Antigen Mucin ist.

**3.** Verfahren nach Anspruch 2, in welchem das Antigen Epiglycanin ist.

**4.** Verfahren nach Anspruch 1, in welchem das Antigen ein Glycoprotein ist.

**5.** Verfahren nach Anspruch 1, in welchem das Antigen ein Glycolipid ist.

**6.** Verfahren nach Anspruch 1, in welchem das Antigen eine T-alpha-Determinante trägt.

**7.** Verfahren nach Anspruch 1, in welchem das Antigen eine T-beta-Determinante trägt.

**8.** Verfahren nach Anspruch 1, in welchem das Antigen eine Tn-Determinante trägt.

**9.** Verfahren nach Anspruch 1, worin das Antigen ein,an einen immunologischen Träger konjugiertes, synthetisches Kohlenhydrat-Hapten umfaßt.

**10.** Verfahren nach Anspruch 9, worin das Hapten eine Determinante, ausgewählt aus der Gruppe, bestehend aus T-alpha-, T-beta- und Tn-Determinanten, trägt.

**11.** Verfahren nach Anspruch 1, in welchem das Antigen ein Polysaccharid ist.

**12.** Verfahren nach Anspruch 1, in welchem das Medium weiterhin ein Lymphokin umfaßt.

**13.** Verfahren nach Anspruch 12, in welchem das Lymphokin IL-2 ist.

**14.** Eine im wesentlichen biologisch reine Kultur sensibilisierter DTH-Effektorzellen, die spezifisch für eine prädeterminierte, Tumor-assoziierte Kohlenhydratdeterminante sind, worin die Effektorzellen durch ein Verfahren gemäß einem der Ansprüche 1 bis 13 sensibilisiert worden sind.

**15.** Verfahren zum Herstellen einer therapeutischen Zusammensetzung, umfassend eine wirksame Menge sensibilisierter DTH-Effektorzellen, die spezifisch für eine prädeterminierte, Tumor-assoziierte Kohlenhydratdeterminante sind, in einem pharmazeutisch annehmbaren Träger, worin die DTH-Effektorzellen durch ein Verfahren gemäß einem der Ansprüche 1 bis 13 sensibilisiert worden sind, welches das Mischen der sensibilisierten DTH-Effektorzellen mit dem Träger in einer an sich bekannten Weise umfaßt.

**16.** Verfahren nach Anspruch 15, in welchem zusätzlich bestrahlte Tumorzellen zu der Zusammensetzung zugemischt werden.

**17.** Verfahren zum Herstellen einer Zusammensetzung, umfassend eine wirksame Menge eines spezifischen, eine Tumor-assoziierte Kohlenhydratdeterminante tragenden Antigens in einem pharmazeutisch annehmbaren Träger zur Verwendung als ein Medikament, welches das Mischen des spezifischen Antigens mit dem Träger in einer an sich bekannten Weise umfaßt.

**18.** Verfahren nach Anspruch 17, in welcher das Antigen ein Mucin ist.

**19.** Verfahren nach Anspruch 18, in welcher das Mucin Epiglycanin ist.

**20.** Verfahren nach Anspruch 17, in welcher die Determinante T-alpha ist.

**21.** Verfahren nach Anspruch 17, in welcher die Determinante T-beta ist.

**22.** Verfahren nach Anspruch 17, in welcher die Determinante Tn ist.

**23.** Verfahren nach einem der Ansprüche 20 bis 22, in welcher das Antigen ein an einen immunologischen Träger konjugiertes` synthetisches Hapten umfaßt.

**24.** Verwendung der Kultur sensibilisierter DTH-Effektorzellen nach Anspruch 14 zum Herstellen eines Medikaments zum Verstärken der zellulären Immunität in einem Säuger.

**25.** Verwendung einer DTH-Effektorzellen sensibilisierenden Menge eines eine spezifische, Tumor-assoziierte Kohlenhydratdeterminante tragenden Kohlenhydratantigens für die Herstellung eines Medikaments zum Verstärken der zellulären Immunität in einem Säuger.

**26.** Verwendung nach Anspruch 25, worin das Antigen ein Mucin ist.

**27.** Verwendung nach Anspruch 26, worin das Mucin Epiglycanin ist.

**28.** Verwendung nach Anspruch 25, worin die Determinante T-alpha ist.

**29.** Verwendung nach Anspruch 25, worin die Determinante T-beta ist.

**30.** Verwendung nach Anspruch 25, worin die Determinante Tn ist.

**31.** Verwendung nach einem der Ansprüche 28 bis 30, worin das Antigen ein an einen immunologischen Träger konjugiertes, synthetisches Hapten umfaßt.

**32.** Verwendung von mit Epiglycanin sensibilisierten DTH-Effektorzellen und bestrahlten Tumorzellen für die Herstellung eines Medikaments zum Induzieren einer DTH-Reaktion in einem Säuger.

**Revendications**
**Revendications pour les Etats contractants suivants : BE, CH, DE, FR, GB, IT, LI, LU, NL, SE**

**1.** Procédé pour amorcer des cellules T mises en jeu dans des réactions d'hypersensibilité retardées (cellules effectrices DTH), qui sont spécifiques d'un déterminant hydrate de carbone associé à une tumeur prédéterminée, pour augmenter leur activité immunologique liée à l'inhibition de la tumeur qui consiste à cultiver dos cellules effectrices DTH dans un milieu contenant une quantité à effet d'amorçage d'un antigène spécifique portant ledit déterminant hydrate de carbone.

**2.** Procédé selon la revendication 1, dans lequel l'antigène est une mucine.

**3.** Procédé selon la revendication 2, dans lequel l'antigène est l'épiglycannine.

**4.** Procédé selon la revendication 1, dans lequel l'antigène est une glycoprotéine.

**5.** Procédé selon la revendication 1, dans lequel l'antigène est un glycolipide

**6.** Procédé selon la revendication 1, dans lequel l'antigène porte un déterminant T-alpha.

**7.** Procédé selon la revendication 1, dans lequel l'antigène porte un déterminant T-bêta.

**8.** Procédé selon la revendication 1, dans lequel l'antigène Porte un déterminant Tn.

**9.** Procédé selon la revendication 1, dans lequel l'antigène comprend un haptène hydrate de carbone synthétique conjugué à un support immunologique.

**10.** Procédé selon la revendication 9, dans lequel l'haptène porte un déterminant choisi parmi l'ensemble comprenant les déterminants T-alpha, T-bêta et Tn.

**11.** Procédé selon la revendication 1, dans lequel l'antigène est un polysaccharide.

**12.** Procédé selon la revendication 1, dans lequel le milieu comprend en outre une lymphokine.

**13.** Procédé selon la revendication 12, dans lequel le lymphokine est IL-2.

**14.** Culture essentiellement pure du point de vue biologique de cellules effectrices DTH amorcées spécifiques d'un déterminant hydrate de carbone associé à une tumeur prédéterminée, dans laquelle les cellules effectrices ont été amorcées par un procédé selon l'une quelconque des revendications 1 à 13.

**15.** Composition thérapeutique comprenant une quantité efficace de cellules effectrices DTH amorcées spécifiques d'un déterminant hydrate de carbone associé à une tumeur prédéterminée, dans un excipient pharmaceutiquement acceptable, dons laquelle les cellules effectrices DTH ont été amorcées par un procédé selon l'une quelconque des revendications 1 à 13.

**16.** Composition selon la revendication 15, comprenant en outre des cellules tumorales irradiées.

**17.** Composition comprenant une quantité efficace d'un antigène spécifique portant un déterminant hydrate de carbone associé à une tumour, dans un excipient pharmaceutiquement acceptable, pour utilisation en tant que médicament.

**18.** Composition selon la revendication 17, dans laquelle l'antigène est une mucine.

**19.** Composition selon la revendication 18, dons laquelle la mucine est l'épiglycannine.

**20.** Composition selon la revendication 17, dans laquelle le déterminant est le déterminant T-alpha.

**21.** Composition selon la revendication 17, dons laquelle le déterminant est le déterminant T-bêta.

**22.** Composition selon la revendication 17, dans laquelle le déterminant est le déterminant Tn.

**23.** Composition selon l'une quelconque des revendications 20 à 22, dans laquelle l'antigène comprend un haptène synthétique conjugué à un support immunologique.

**24.** Utilisation d'une culture de cellules effectrices DTH amorcées selon la revendication 14, pour préparer un médicament destiné à augmenter l'immunité cellulaire chez un mammifère.

**25.** Utilisation d'une quantité à effet d'amorçage de cellules effectrices DTH d'un antigène hydrate de carbone portant un déterminant hydrate de carbone associé à une tumeur spécifique, pour préparer un médicament destiné à augmenter l'immunité cellulaire chez un mammifère.

**26.** Utilisation selon la revendication 25, dans laquelle l'antigène est une mucine.

**27.** Utilisation selon la revendication 26, dans laquelle la mucine est l'épiglycannine.

**28.** Utilisation selon la revendication 25, dans laquelle le déterminant est le déterminant T-alpha.

**29.** Utilisation selon la revendication 25, dans laquelle le déterminant est le déterminant T-bêta.

**30.** Utilisation selon la revendication 25, dans laquelle le déterminant est le déterminant Tn.

**31.** Utilisation selon l'une quelconque des revendications 28 à 30, dans laquelle l'antigène comprend un haptène synthétique conjugué à un support immunologique.

**32.** Utilisation de cellules effectrices DTH amorcées par l'épiglycannine et de cellules tumorales irradiées, pour préparer un médicament destiné à provoquer une réaction DTH chez un mammifère.

**Revendications pour l'Etat contractant suivant : AT**

**1.** Procédé pour amorcer des cellules T mises en jeu dans des réactions à,hypersensibilité retardées (cellules effectrices DTH), qui sont spécifiques d'un déterminant hydrate de carbone associé à une tumeur prédéterminée, pour augmenter leur activité immunologique liée à l'inhibition de la tumeur qui consiste à cultiver des cellules effectrices DTH dans un milieu contenant une quantité à effet d'amorçage d'un antigène spécifique portant ledit déterminant hydrate de carbone.

**2.** Procédé selon la revendication 1, dans lequel l'antigène est une mucine.

**3.** Procédé selon la revendication 2, dans lequel l'antigène est l'épiglycannine.

**4.** Procédé selon la revendication 1, dans lequel l'antigène est une glycoprotéine.

**5.** Procédé selon la revendication 1, dans lequel l'antigène est un glycolipide.

**6.** Procédé selon la revendication 1, dans lequel l'antigène porte un déterminant T-alpha.

**7.** Procédé selon la revendication 1, dans lequel l'antigène Porte un déterminant T-bêta.

**8.** Procédé selon la revendication 1, dans lequel l'antigène porte un déterminant Tn.

**9.** Procédé selon la revendication 1, dans lequel l'antigène comprend un haptène hydrate de carbone synthétique conjugué à un support immunologique.

**10.** Procédé selon la revendication 9, dans lequel l'haptène porte un déterminant choisi parmi l'ensemble comprenant les déterminants T-alpha, T-bêta et Tn.

**11.** Procédé selon la revendication 1, dans lequel l'antigène est un polysaccharide.

**12.** Procédé selon la revendication 1, dans lequel le milieu comprend en outre une lymphokine.

**13.** Procédé selon la revendication 12, dons lequel la lymphokine est IL-2.

**14.** Culture essentiellement pure du point de vue biologique de cellules effectrices DTH amorcées spécifiques d'un déterminant hydrate de carbone associé à une tumeur prédéterminée, dans laquelle les cellules effectrices ont été amorcées par un procédé selon l'une quelconque des revendications 1 à 13.

**15.** Procédé pour préparer une composition thérapeutique comprenant une quantité efficace de cellules effectrices DTH amorcées spécifiques d'un déterminant hydrate de carbone associé à une tumeur prédéterminée, dans un excipient pharmaceutiquement acceptable, dans laquelle les cellules effectrices DTH ont été amorcées par un procédé selon l'une quelconque des revendications 1 à 13, qui consiste à mélanger les cellules effectrices DTH amorcées à l'excipient d'une manière connue en soi.

**16.** Procédé selon la revendication 15, dans lequel des cellules tumorales irradiées sont en outre mélangées à la composition.

**17.** Procédé pour préparer une composition comprenant une quantité efficace d'un antigène spécifique portant un déterminant hydrate de carbone associé à une tumeur, dans un excipient pharmaceutiquement acceptable, pour utilisation on tant que médicament, qui consiste à mélangor l'antigène spécifique à l'excipient d'une manière connue en soi.

**18.** Procédé selon la revendication 17, dons lequel l'antigène est une mucine.

**19.** Procédé selon la revendication 18, dans lequel la mucine est l'épiglycannine.

**20.** Procédé selon la revendication 17, dans lequel le déterminant est le déterminant T-alpha.

**21.** Procédé selon la revendication 17, dans lequel le déterminant est le déterminant T-bêta.

**22.** Procédé selon la revendication 17, dans lequel le déterminant est le déterminant Tn.

**23.** Procédé selon l'une quelconque des revendications 20 à 22, dans lequel l'antigène comprend un haptène synthétique conjugué à un support immunologique.

**24.** Utilisation d'une culture de cellules effectrices DTH amorcées selon la revendication 14, pour préparer un médicament destiné à augmenter l'immunité cellulaire chez un mammifère.

16

**25.** Utilisation d'une quantité à effet d'amorçage de cellules effectrices DTH d'un antigène hydrate de carbone portant un déterminant hydrate de carbone associé à une tumeur spécifique, pour préparer un médicament destiné à augmenter l'immunité cellulaire chez un mammifère.

**26.** Utilisation selon la revendication 25, dans laquelle l'antigène est une mucine.

**27.** Utilisation selon la revendication 26, dans laquelle la mucine est l'épiglycannine.

**28.** Utilisation selon la revendication 25, dans laquelle le déterminant est le déterminant T-alpha.

**29.** Utilisation selon la revendication 25, dans laquelle le déterminant est le déterminant T-bêta.

**30.** Utilisation selon la revendication 25, dans laquelle le déterminant est le déterminant Tn.

**31.** Utilisation selon l'une quelconque des revendications 28 à 30, dans laquelle l'antigène comprend un haptène synthétique conjugué à un support immunologique.

**32.** Utilisation de cellules effectrices DTH amorcées par l'épiglycannine et de cellules tumorales irradiées, pour préparer un médicament destiné à provoquer une réaction DTH chez un mammifère.

## F I G. I.

# FIG. 2.

## FIG. 3.

FIG. 4.

CELL SURFACE PHENOTYPE OF DTH CELL

| | Non tr | a Thy | a Lyt I | aLyt 2 | C' | Ag |
|---|---|---|---|---|---|---|
| % Lysis<br>c Ab tr. | 1.0 | 40 | 20 | 30 | 5 | |

SYSTEMIC TRANSFER OF DTH    CH= 4hr     *FIG. 5.*

TO 88

EP 0 313 569 B1

FIG. 6.

INHIBITION OF LOCAL TUMOR GROWTH
(to–7i)

FOOT PAD SWELLING

□ PRIMED + TA3Hα     + NORMAL + TA3Hα

*FIG. 7.*

ANTI TN DTH
(TO-46)

DTH

SD

☒ USED

EP 0 313 569 B1

FIG. 8.